# EUROPEAN PATENT SPECIFICATION

(11) **EP 3 444 615 B1**
(45) Date of publication and mention of the grant of the patent: **08.09.2021**
(21) Application number: 17186234.5
(22) Date of filing: 15.08.2017
(51) Int. Cl.: G01N 33/72

(54) **METHODS AND COMPOSITIONS FOR DIAGNOSTIC EVALUATION AND THERAPEUTIC CORRECTION OF THE NO-HAEM SIGNALLING PATHWAY(S) IN ANIMALS AND HUMANS**
VERFAHREN UND ZUSAMMENSETZUNGEN ZUR DIAGNOSTISCHEN BEURTEILUNG UND THERAPEUTISCHEN KORREKTUR DES NO-HAEM-SIGNALWEGS BEI TIEREN UND MENSCHEN
PROCÉDÉS ET COMPOSITIONS POUR L'ÉVALUATION DIAGNOSTIQUE ET LA CORRECTION THÉRAPEUTIQUE DES VOIES DE SIGNALISATION NON HAÈME CHEZ L'HOMME ET LES ANIMAUX

(43) Date of publication of application: 20.02.2019
(73) Proprietor: Freiberg Instruments GmbH, 09599 Freiberg (DE)
(72) Inventor: KLESCHYOV, Andrei L., 55122 Mainz (DE)
(74) Representative: Patentanwälte Dr. Keller, Schwertfeger Partnerschaft mbB

(56) References cited:
- EP-A1- 1 780 274
- WO-A1-98/34955
- WO-A2-2004/087212
- US-A1- 2005 112 717
- US-A1- 2005 227 308
- US-B1- 6 291 424
- STAMLER J S ET AL: "Biochemistry of nitric oxide and its redox-activated forms", SCIENCE, AMERICAN ASSOCIATION FOR THE ADVANCEMENT OF SCIENCE, vol. 258, 18 December 1992 (1992-12-18), pages 1898-1902, XP003023011, ISSN: 0036-8075, DOI: 10.1126/SCIENCE.1281928
- KLESCHYOV ANDREI L FREIBERG INSTRUMENTS GMBH LAB BIOPHYS D-09599 FREIBERG GERMANY ET AL: "The NO-heme signaling hypothesis", FREE RADICAL BIOLOGY AND MEDICINE, ELSEVIER INC, US , vol. 112 31 October 2017 (2017-10-31), pages 544-552, XP009501597, ISSN: 0891-5849, DOI: 10.1016/J.FREERADBIOMED.2017.08.025 Retrieved from the Internet: URL:http://www.sciencedirect.com/science/a rticle/pii/S0891584917307414

## Description

### Background of the invention

Nitric oxide (NO) and NO-related biomolecules produced by the constitutive NO synthase isoforms (NOS1 and NOS3) has been recognized as important signalling species in cardiovascular, nervous, gastrointestinal, and virtually all other physiological systems. The exact nature and specific activity of these potentially multiple NO-containing biomolecules are not yet fully elucidated. However, it is well known, that large quantities of free-NO produced by the inducible NO synthase isoform (NOS2) are extremely toxic and utilized by immune system for the elimination of infectious pathogens, parasites and tumour cells (Wallis JP, Nitric oxide and blood: a review. Transfus. Med., 15(1):1-11 (2005); Bogdan, C. Nitric oxide synthase in innate and adaptive immunity: an update. Trends in immunology 36, 161-178, (2015); Lehnert et al., The Handbook of Porphyrin Science, Kadish et al., World Scientific: NJ, Vol. 14, pp 1-247, 2011; Roncaroli et al., New Features in the Redox Coordination Chemistry of Metal Nitrosyls, Coord. Chem. Rev., 251, 1903-1930, (2007); Ignarro, L. J. Nitric oxide. A novel signal transduction mechanism for transcellular communication. Hypertension 16, 477-483 (1990)).

All NOSs are haem-dependent homodimer enzymes which synthesize NO and/or NO-like biomolecules from the amino acid L-arginine. The activity of NOS is regulated by numerous cofactors and protein-protein interactions. The major down-stream target of the constitutive NOS1/NOS3 is thought to be also a haem-dependent enzyme - soluble guanylyl cyclase (sGC). The sGC produces the second messenger, cyclic GMP which regulates critical physiological functions (vascular tone, platelet aggregation, cell growth, neural transmission etc.). Additionally, there are many other NOS-dependent (but sGC-independent) signalling pathways, which involve the S-nitrosative modification of many enzymes, receptors, ionic channels, membrane transporters, transcriptional factors etc.

It is fundamentally unknown, how these multiple NOS1/NOS3-dependent signalling pathways are coordinated/selectively regulated by the simple molecule NO. The lack of this knowledge severely limits the practical implications of the many discoveries made in the NOS field. Clearly, free NO (small, uncharged, reactive and potentially toxic molecule) is unable to perform this complex and diverse signalling function(s).

According to the conventional view, all NOS isoforms produce free-NO (gas) which diffuses within and between cells until it meets/reacts with its biological targets (haem-sGC or selective cysteine residues of proteins etc.). However, in physiological environment NO must have countless nonspecific and (often) deleterious reactions with other biomolecules (transitional metals, molecular oxygen, free radicals, lipids, peptides, DNA etc.). How NO manages to recognize the specific targets is unknown. It has been previously proposed that low molecular weight S-nitrosothiols (such as S-nitrosoglutathione or S-nitrosocysteine) are major mediators of the NO-related bioactivity (Stamler, J. S., Singel, D. J. & Loscalzo, J. Biochemistry of nitric oxide and its redox-activated forms. Science 258, 1898-1902 (1992)). However, the regulation mechanism of the S-nitrosothiol formation, transportation, transnitros(yl)ation and decomposition to NO (at sGC), are not understood.

Despite 30 years of intensive research efforts and more than 150.000 publications, the valuable NO- or S-nitrosothiol-based diagnostics and therapeutics are not appeared on the market. The possible misconcept of the free-NO signalling paradigm might be a reason for the lack of practical outcome in NOS/NO-studies. Revealing the physiological mediators/mechanisms of NOS1/NOS2 bioactivity is a prerequisite for the development of new diagnostic tools and drugs.

Canonically, NO freely diffuses through the (inter)cellular milieu and binds to its major target, the prosthetic ferrohaem group of sGC, resulting in several hundred-fold enzyme activation. At the same time, different ferrohaem-proteins, such as haemoglobin (Hb) and myoglobin (Mb) are well known to competitively scavenge NO and prevent the sGC activation (Mittal, C. K., Arnold, W. P. & Murad, F. Characterization of protein inhibitors of guanylate cyclase activation from rat heart and bovine lung. The Journal of biological chemistry 253, 1266-1271 (1978); Martin, W., Villani, G. M., Jothianandan, D. & Furchgott, R. F. Selective blockade of endothelium-dependent and glyceryl trinitrate-induced relaxation by hemoglobin and by methylene blue in the rabbit aorta. The Journal of pharmacology and experimental therapeutics 232, 708-716 (1985); Palmer, R. M., Ferrige, A. G. & Moncada, S. Nitric oxide release accounts for the biological activity of endothelium-derived relaxing factor. Nature 327, 524-526, doi:10.1038/327524a0 (1987); Ignarro, L. J. Nitric oxide. A novel signal transduction mechanism for transcellular communication. Hypertension 16, 477-483 (1990)).

Several NOS/NO-related findings, however, were difficult to reconcile with this widely accepted view. Thus, it was found out that nitrite, which is present in tissues in low µM concentrations, and normally inactive, becomes an important source of NO/sGC-bioactivity under hypoxic conditions. The current explanation of this finding is that nitrite undergoes one-electron reduction by deoxy-Fe2+-haemoproteins (mainly by Hb and Mb) forming the NO-releasing NO-ferri(Fe3+)haem species. The unanswered question is how NO produced by this way avoids scavenging by the huge molar excess of Hb/Mb which are present just in the site of hypothetical NO production? A similar question arises regarding the intra-erythrocyte NOS3, which was found to control the vascular tone and platelet aggregation. Further, recent studies have questioned the role of NO in the vasodilator activity of the widely-used drug, nitroglycerin (GTN). Being a powerful vasodilator and sGC activator (pM to nM range), NTG is a weak NO donor elevating vascular NO levels only at supra-pharmacological concentrations (µM to mM range) (Kleschyov, A. L. et al. Does nitric oxide mediate the vasodilator activity of nitroglycerin? Circ. Res. 93, e104-112 (2003); Nunez, C. et al. Discrepancies between nitroglycerin and NO-releasing drugs on mitochondrial oxygen consumption, vasoactivity, and the release of NO. Circulation research 97, 1063-1069 (2005)). These inconsistencies, as well as the lack of important practical outcomes of new synthetic NO-donors urge to search for alternative mediators of the NOS-related signalling network.

The currently accepted free NO/sCG-signalling paradigm of the prior art implies the non-directional diffusion of NO from the site of its production to its major target, ferrohaem-sGC. This model seems to be unrealistic for several reasons: (1) NO has many other targets, including countless haem and nonhaem iron complexes, which are present in cells in µM to mM concentrations and have comparable reaction rate constants for NO, as sGC does (Ford, P. C. Reactions of NO and nitrite with heme models and proteins. Inorganic chemistry 49, 6226-6239 (2010); Toledo, J. C., Jr. & Augusto, O. Connecting the chemical and biological properties of nitric oxide. Chemical research in toxicology 25, 975-989 (2012)). (2) NO is much more soluble in lipids than in water. Therefore, NO accumulates in biological membranes and hydrophobic cores of proteins where it has accelerated reaction with molecular oxygen. (3) NO rapidly partitions into gas phase (Ford, P. C. Reactions of NO and nitrite with heme models and proteins. Inorganic chemistry 49, 6226-6239 (2010)). (4) NO rapidly reacts with the primary biological oxidant, superoxide; this reaction not only consumes the bioactive NO, but forms the toxic species, peroxynitrite (Beckman, J. S., Beckman, T. W., Chen, J., Marshall, P. A. & Freeman, B. A. Apparent hydroxyl radical production by peroxynitrite: implications for endothelial injury from nitric oxide and superoxide. Proceedings of the National Academy of Sciences of the United States of America 87, 1620-1624 (1990). (5) NO rapidly reacts with oxy-Hb to form nitrate (Ford, P. C. Reactions of NO and nitrite with heme models and proteins. Inorganic chemistry 49, 6226-6239, doi:10.1021/ic902073z (2010)) and with ceruloplasmin to form nitrite (Shiva, S. et al. Ceruloplasmin is a NO oxidase and nitrite synthase that determines endocrine NO homeostasis. Nature chemical biology 2, 486-493 (2006)); paracrine and endocrine NO signalling function is severely compromised. (6) NO at nanomolar concentrations inhibits cytochrome C oxidase (Brown, G. C. & Cooper, C. E. Nanomolar concentrations of nitric oxide reversibly inhibit synaptosomal respiration by competing with oxygen at cytochrome oxidase. FEBS letters 356, 295-298 (1994)). (7) NO must rapidly (especially at high concentration within the NOS cleft and biological membranes) react with molecular oxygen to form, among other species, N₂O₃, which is a strong but non-selective toxic/genotoxic nitrosative agent (Liu, X., Miller, M. J., Joshi, M. S., Thomas, D. D. & Lancaster, J. R., Jr. Accelerated reaction of nitric oxide with O2 within the hydrophobic interior of biological membranes. Proceedings of the National Academy of Sciences of the United States of America 95, 2175-2179 (1998)). These inherent NO limitations imply that the recruitment of free-NO gas for a sustained regulation of physiological functions contradicts to the principle of biological expediency - it would be wasteful, stochastic and even hazardous. However, the NO diffusion model is clearly reasonable for the acute NO/sGC signalling and for the innate-immunity NOS2/NO function.

The currently used methods in the prior art are mostly indirect and unable to discriminate between tissue NO and NO-haem species, especially under basal physiological conditions. The basal levels of NO, reported in the literature, vary greatly (up to 10⁶). Despite 30 years of research, the quantitative link between NO and physiological effect is lacking. In conclusion, the widely accepted free NO diffusion signalling paradigm of the prior art cannot adequately describe the NOS-dependent signalling network.

Early studies on the purified sGC preparations have shown that different paramagnetic NO-ferrohaem-proteins (NO-Hb; NO-Mb; NO-catalase) are efficient sGC activators (Craven, P. A. & DeRubertis, F. R. Restoration of the responsiveness of purified guanylate cyclase to nitrosoguanidine, nitric oxide, and related activators by heme and hemeproteins. Evidence for involvement of the paramagnetic nitrosyl-heme complex in enzyme activation. The Journal of biological chemistry 253, 8433-8443 (1978); Craven, P. A., DeRubertis, F. R. & Pratt, D. W. Electron spin resonance study of the role of NO catalase in the activation of guanylate cyclase by NaN3 and NH2OH. Modulation of enzyme responses by heme proteins and their nitrosyl derivatives. The Journal of biological chemistry 254, 8213-8222 (1979)). It has been postulated that the mechanism of activation of the isolated sGC may involve the transfer of NO-ferrohaem group from the NO-ferrohaem-proteins to sGC (Ignarro, L. J., Adams, J. B., Horwitz, P. M. & Wood, K. S. Activation of soluble guanylate cyclase by NO-hemoproteins involves NO-heme exchange. Comparison of heme-containing and heme-deficient enzyme forms. The Journal of biological chemistry 261, 4997-5002 (1986)). It was found that NO-ferrohaem binds to sGC stronger than ferrohaem does (K_{d} values: 1-5 nM and 100-200 nM, respectively). In contrast to NO-ferrohaem, bare ferrohaem inhibits the basal activity of isolated sGC (Wolin, M. S., Wood, K. S. & Ignarro, L. J. Guanylate cyclase from bovine lung. A kinetic analysis of the regulation of the purified soluble enzyme by protoporphyrin IX, heme, and nitrosyl-heme. The Journal of biological chemistry 257, 13312-13320 (1982); Ignarro, L. J., Wood, K. S. & Wolin, M. S. Activation of purified soluble guanylate cyclase by protoporphyrin IX. Proceedings of the National Academy of Sciences of the United States of America 79, 2870-2873 (1982); Ignarro, L. J., Adams, J. B., Horwitz, P. M. & Wood, K. S. Activation of soluble guanylate cyclase by NO-hemoproteins involves NO-heme exchange. Comparison of heme-containing and heme-deficient enzyme forms. The Journal of biological chemistry 261, 4997-5002 (1986); Ohlstein, E. H., Wood, K. S. & Ignarro, L. J. Purification and properties of heme-deficient hepatic soluble guanylate cyclase: effects of heme and other factors on enzyme activation by NO, NO-heme, and protoporphyrin IX. Archives of biochemistry and biophysics 218, 187-198 (1982)).

Haem plays a crucial role in the assembly and stabilization of various haemoproteins (both homo-dimers and homo-tetramers), including Hb and Mb (both are haem-His liganded), catalase (haem-Tyr liganded) and NOS (haem-Cys liganded) (Hargrove, M. S., Barrick, D. & Olson, J. S. The association rate constant for heme binding to globin is independent of protein structure. Biochemistry 35, 11293-11299 (1996); List, B. M. et al. Characterization of bovine endothelial nitric oxide synthase as a homodimer with down-regulated uncoupled NADPH oxidase activity: tetrahydrobiopterin binding kinetics and role of haem in dimerization. The Biochemical journal 323 (Pt 1), 159-165 (1997); Smith, A. T. et al. Functional divergence of heme-thiolate proteins: a classification based on spectroscopic attributes. Chemical reviews 115, 2532-2558 (2015)). The enzymatic formation of NO-ferrohaem often results in a destabilization of the haemoproteins and release/transfer of NO-ferrohaem to another acceptor (Ignarro, L. J., Adams, J. B., Horwitz, P. M. & Wood, K. S. Activation of soluble guanylate cyclase by NO-hemoproteins involves NO-heme exchange. Comparison of heme-containing and heme-deficient enzyme forms. The Journal of biological chemistry 261, 4997-5002 (1986)). In accordance with this view, in the cell-free system, Hb/Mb/catalase do not transfer ferrohaem to apo-sGC, but NO-Hb/NO-Mb/NO-catalase readily transfer the NO-ferrohaem species to apo-sGC (Ignarro, L. J., Adams, J. B., Horwitz, P. M. & Wood, K. S. Activation of soluble guanylate cyclase by NO-hemoproteins involves NO-heme exchange. Comparison of heme-containing and heme-deficient enzyme forms. The Journal of biological chemistry 261, 4997-5002 (1986)). The mobilization of NO-ferrohaem also may occur from cytochrome P450, which is, like NOS, a haem-Cys liganded protein (Kim, Y. M. et al. Loss and degradation of enzyme-bound heme induced by cellular nitric oxide synthesis. The Journal of biological chemistry 270, 5710-5713 (1995)).

The paramagnetic NO-ferrohaem species can be generated both enzymatically from amines and via an ordinary chemistry, such as: (1) reaction of NO with penta-coordinated ferrohaem (kₒₙ ∼ 10⁸-10¹⁰ M⁻¹s⁻¹) (Ford, P. C. Reactions of NO and nitrite with heme models and proteins. Inorganic chemistry 49, 6226-6239 (2010)); (2) reaction of NO with penta-coordinated ferrihaem (kₒₙ ∼ 10³-10⁵ M⁻¹s⁻¹) followed by the reduction with nucleophiles or excess of NO 23; (3) reaction of ferrihaem with nitroxyl (HNO)(kₒₙ ∼10⁷-10⁸ M⁻¹s⁻¹) (Bari, S. E., Marti, M. A., Amorebieta, V. T., Estrin, D. A. & Doctorovich, F. Fast nitroxyl trapping by ferric porphyrins. Journal of the American Chemical Society 125, 15272-15273 (2003); Speelman, A. L. & Lehnert, N. Heme versus non-heme iron-nitroxyl {FeN(H)O}(8) complexes: electronic structure and biologically relevant reactivity. Accounts of chemical research 47, 1106-1116 (2014)); (4) reaction of nitrite with ferrohaem generating NO-ferrihaem which transfers NO to a neighbouring ferrohaem or undergoes autoreduction (Lundberg, J. O. et al. Nitrate and nitrite in biology, nutrition and therapeutics. Nature chemical biology 5, 865-869 (2009); Ford, P. C. Reactions of NO and nitrite with heme models and proteins. Inorganic chemistry 49, 6226-6239 (2010); Reutov, V. P. Biochemical predetermination of the NO synthase and nitrite reductase components of the nitric oxide cycle. Biochemistry. Biokhimiia 64, 528-542 (1999)). It should be noted, however, that despite the common reaction product is NO-ferrohaem, each mechanism may be linked to a defined intracellular compartment and, thus be responsible for a specific signalling. For example, in the heart, HNO donors (which react with ferrihaem) are known to exert the calcitonin gene-related peptide-mediated positive inotropic effect, the activity which is not reproduced by NO donors (Paolocci, N. et al. Nitroxyl anion exerts redox-sensitive positive cardiac inotropy in vivo by calcitonin gene-related peptide signaling. Proceedings of the National Academy of Sciences of the United States of America 98, 10463-10468 (2001)).

The NO-haem signalling must be intrinsically linked to the intracellular, regulatory, exchangeable haem pool. This ill-defined, weakly-bound haem is thought to control the key cellular functions through the regulation of genes, kinases, ion channels and receptors; it is thought to be highly heterogeneous in terms of distribution, kinetics, dynamics, protein carriers, oxidation state and attached small ligands; the average levels of the regulatory haem are about 100 nM (Ponka, P. Tissue-specific regulation of iron metabolism and heme synthesis: distinct control mechanisms in erythroid cells. Blood 89, 1-25 (1997); Smith, A. G., Raven, E. L. & Chernova, T. The regulatory role of heme in neurons. Metallomics: integrated biometal science 3, 955-962 (2011); Furuyama, K., Kaneko, K. & Vargas, P. D. Heme as a magnificent molecule with multiple missions: heme determines its own fate and governs cellular homeostasis. The Tohoku journal of experimental medicine 213, 1-16 (2007)). Studies have shown that most cell types synthesize their own haem, which then enters the regulatory pool and rapidly undergoes the breakdown (Lodola, A. & Jones, O. T. Evidence for a rapidly turned over pool of haem in isolated hepatocytes. FEBS letters 90, 250-254 (1978); Takeda, T. A., Mu, A., Tai, T. T., Kitajima, S. & Taketani, S. Continuous de novo biosynthesis of haem and its rapid turnover to bilirubin are necessary for cytoprotection against cell damage. Scientific reports 5, 10488 (2015)). It is likely that the NO-ferrohaem streamlet is an important part of the regulatory haem pool turnover.

The rate/regulation of haem synthesis in NOS-expressing cells was not specifically studied. The activity of ALAS (the rate-limiting enzyme in haem biosynthesis) in different non-erythroid animal tissues were reported to be between 5.6 and 605 nmol/g tissue x hr (Jaronczyk, K. et al. The source of heme for vascular heme oxygenase II: de novo heme biosynthesis in rat aorta. Canadian journal of physiology and pharmacology 82, 218-224 (2004), and the references therein). Therefore, it may be calculated that the flux of newly synthesized haem in nonerythroid cells might be between 1 and 200 nM/s. Interestingly, these values roughly correspond to the hypothetical NO flux in neuronal cells (calculations were based on the sGC response) (Wood, K. C. et al. Picomolar nitric oxide signals from central neurons recorded using ultrasensitive detector cells. The Journal of biological chemistry 286, 43172-43181 (2011)). The proposed NO-haem signalling concept implies that, to be efficient and save, the fluxes of both NO and haem must match each another. This ideal situation is likely may be adjusted for the basal constitutive NOS1 and NOS3 activities. However, the (over)stimulation of NOS1/NOS3 may lead to the NO/haem uncoupling and contribute to the tissue damage.

The term of NO-haem integrates multiple (yet potentially interconvertible) species. Such NO-haem variability is a function of (1) iron oxidation state and (2) small ligand coordinated at the 6th (trans to NO) position. As both factors dictate the haem/NO reactivity (Ford, P. C., Fernandez, B. O. & Lim, M. D. Mechanisms of reductive nitrosylation in iron and copper models relevant to biological systems. Chemical reviews 105, 2439-2455 (2005)), they must be critical for the NO-haem binding and, eventually, signalling function. Among candidates for such haem small ligands are amino acids, nucleotides and, especially, gaseous molecules (NO, CO, H2S, HCN etc.). Interestingly, NO, CO and H2S are known to modulate the each other's bioactivity (Hartsfield, C. L. Cross talk between carbon monoxide and nitric oxide. Antioxidants & redox signalling 4, 301-307 (2002)). Thus, it is quite possible that the exchangeable haem may be a point where NO, CO and H2S (HS-) interact.

The above described data were obtained from independent sources and were never put together in a physiological/pharmacological context. What is important to realize is that the self-same active ternary NO-ferrohaem-sGC complex can be formed in two ways: (1) reaction of NO with ferrohaem-sGC and (2) reaction of preformed NO-ferrohaem with haem-free sGC. During last 30 years, the research efforts have been focused, solely, on the first possibility, implying that in vivo sGC is a ferrohaem-containing enzyme and free NO is an endogenous signalling species. The second possibility implying that mobile NO-ferrohaem might be the endogenous activator of sGC has never been considered in the literature. Because the bound between NO and haem is very strong, NO-haem can behave as a signalling entity. Because the bare ferrohaem binds to sCG weakly and inhibits the basal sGC activity, it can be regarded as the sCG-receptor antagonist. Because, NO-ferrohaem binds to sGC firmly and activates the enzyme, it can be regarded as the sCG-receptor agonist. The present invention is based on the idea that mobile/exchangeable NO-haem species are natural sGC-activating signalling mediators.

Despite initial hope and large investments, the 30-years of NO-research efforts did not result in development of important diagnostic tests or drugs. A likely reason of this failure is the imperfection of the free NO-gas signalling model. The presented novel NO-haem signalling concept corrects the presently accepted NO-gas paradigm and opens new diagnostic and therapeutic perspectives. The inventors predict that: (1) determination of mobilized NO-haem in biological fluids may have diagnostic and prognostic value and (2) therapeutic manipulations on the NO-haem signalling pathway may comprise a novel approach for the prevention and treatment of widespread diseases.

WO 98/34955 A1 describes the use of nitrosylated ferrous heme based preparations in the treatment of cardiovascular diseases, inflammation and respiratory diseases. The administration of a nitrosyl-heme-containing donor of NO is suggested. Preferably, the nitrosyl-heme-containing donor of NO is nitrosylhemoglobin. A nitrosated and/or nitrosylated heme protein is described in US 6,291,424 B1.

WO 2004/087212 A2 describes the use of nitric oxide or a nitric oxide donor in combination with a glucocorticoid for the treatment of inflammation.

US 2005/011 2717 A1 describes methods for measuring nitric oxide synthase activity comprising (a) a solution comprising a nitric oxide synthase and a suitable substrate under conditions which allow synthesis of nitric oxide; (b) adding guanylyl cyclase and GTP under conditions which allow synthesis of cGMP; and (c) measuring cGMP.

However, the detection of nitric oxide synthase (NOS)/soluble guanylyl cyclase (sGC) activity in a biological sample, based on the determination of the presence and/or quantity of nitrosylated ferrous heme (NO-ferrohaem) in an isolated biological sample has not yet been described. Against this background, it is the object of the present invention to provide methods for the detection of a malfunction of the nitric oxide synthase (NOS)/soluble guanylyl cyclase (sGC) pathway in a biological sample.

This problem is solved by a method with the features of claim 1. Preferred embodiments are part of the dependent claims.

Based on the physico-chemical and biochemical properties of NO-haem, the inventors predict a wide range of bioactivity of the NO-haem entity (both alone and bound to a specific carrier). This bioactivity is due to the non-covalent binding of NO-haem entity to sGC (and other proteins having H-NOX domain) and S-(N-) nitrosation of selective groups of biomolecules. In the present invention, the general term NO-haem designates nitric oxide (NO) bound to haem (iron-protoporphyrin-IX). More specifically, NO-ferrohaem designates NO-(Fe²⁺)haem, while its one-electron oxidation product, NO-ferrihaem designates NO-(Fe³⁺)haem.

The inventor has found that NO-ferrohaem is sufficiently stable and elicits sGC-related bioactivity. This bioactivity is likely due to direct transfer of NO-ferrohaem unity, but not due to release free NO or free nitrosonium cation (NO+). By contrast, NO-ferrihaem is less stable and elicits sGC-independent bioactivity, probably due to the transfer of NO+ to thiol and amino groups of critical biomolecules.

NO-ferrohaem /NO-ferrihaem of the invention designates the first level of discrimination, the second level of discrimination relates to a ligand that is attached to iron at the 6^{th} vacant (trans to NO) position. These ligands may be different for ferrohaem and ferrihaem, and significantly affect the electronic configuration and bioactivity of NO-haem. Among candidates for such small ligands are amino acids, nucleotides, some drugs and gaseous molecules (e.g. NO, CO, H₂S, HCN). Preferred ligands in the NO-haem of the invention are NO, CO and H₂S.

The inventors state that mobile/exchangeable NO-haem (alone or in association with a carrier) may account for most of the NOS-related physiological phenomena, previously attributed to free NO. Preferred NO-haem carriers are albumin, lipoproteins, heat shock protein 90, haemoglobin, myoglobin, catalase, peptides and many other, yet unidentified NO-haem binding biomolecules. The NO-haem could be incorporated into liposomes, erythrocytes, some specific cell types and cell-derived exosomes (microparticles). The inventors substantiate that the basal activity of NOS1 and NOS2 is mediated by NO-haem species but not NO-gas or low molecular weight S-nitrosothiols. Accordingly, the inventors propose to switch the focus of the search for new diagnostics/drugs from NO-gas to mobile NO-haem - a key NOS-related bioactive species in vivo.

The bioactive NO-haem species of the invention can be identified in biological fluids and be of diagnostic importance in cardiovascular, thrombotic, neuro-degenerative, inflammatory and malignant diseases - the major causes of premature death worldwide. The local intracellular NO-ferrohaem/haem (sGC agonist/antagonist) ratio is regarded to be critical for the compartmental sGC activation state. Supply of a small amount of NO-ferrohaem to a specific sGC-compartment would result in a strong local activation of sGC. This prediction is partially supported the recently published data on the NO-dependent inhibition of the hsp90-driven haem insertion to a variety of haemoproteins, but activation of haem (likely NO-haem) insertion to sGC (Ghosh, A. et al. Nitric oxide and heat shock protein 90 activate soluble guanylate cyclase by driving rapid change in its subunit interactions and heme content. J Biol Chem 289, 15259-15271 (2014)).

The unexpected findings of the invention demonstrating that a mobile (carrier-bound, exchangeable) NO-ferrohaem can exert the NOS/sGC-related bioactivity in human and animal tissues are prerequisites for the development of new diagnostic and pharmaceutical applications. It must be stressed that NO ferrohaem is the active principle per se and should not be considered as a donor of free NO. Furthermore, as endogenous signalling species, NO-haem can be formed by several enzymatic systems without participation of free NO. A carrier exchangeable NO-haem species appears to play a key mediatory role in multiple NOS-signalling pathways, currently attributed to free NO by the prior art.

The NO-ferrohaem is an efficient sGC activator due to low nM affinity of NO-ferrohaem-entity to the binding site on sGC. It therefore very efficiently competes with free ferrohaem, which has a 2 orders lower affinity for the binding site on sGC. The NO-ferrohaem of the invention is also a thermodynamically stabile species, which allows the transportation of NO-ferrohaem as a functional entity, without losing NO. The NO-ferrohaem has multiple protein binding sites with differential affinity, and it does not produce deleterious reactive species. The inventor also found that the rate of NO-group synthesis by constitutive NOSs and the rate of new haem synthesis/supply essentially match each another under basal/physiological conditions. The overstimulation of constitutive NOS isoforms, induction of NOS2 or inhibition of haem synthesis may lead to an "NO/haem uncoupling state", increase in the NO/NO-haem ratio, tissue damage and development of number of diseases.

The present invention allows to discriminate between tissue NO and mobile NO-haem species, preferably under basal physiological conditions in animals and human beings.

In addition to sGC signalling, mobile NO-ferrohaem of the invention and its redox sibling, NO-ferrihaem can contribute to selective S-(N-) protein nitrosation-related signalling. Due to the electron charge transfer toward iron, the NO-ferrihaem exist as ⁺NO-Fe²⁺haem species, which is capable to trans-nitrosate thiols and certain amines. The site-specific S-(N-) nitrosation is achieved through the recognition of the selective target by an NO-haem protein carrier of the invention, as well as by the protoporphyrin-IX structure itself. Such specific S-(N-) protein nitrosation is impossible in the case of free NO or NO-derived reactive species of the prior art. Recognition of the fact that a protein carrier-haem-NO+ complex is the optimal physiological tool for S- (N-)nitrosation of selective protein residues becomes a basis for development of new diagnostics and therapeutics.

In a further aspect of the present invention, NO-haem is coupled to a specific carrier to convey NO-haem species to a specific cellular compartment; this principle may allow the pleiotropic NO-haem function, depending on the carrier. Preferred transport carriers are compounds that binds and deliver the bioactive NO-haem species to a defined target cells/compartments. Further preferred carrier candidates are different intracellular haem-binding proteins and transmembrane transporters, which are known to bind or release haem in a redox-, pH- and conformation-dependent manner (Schultz, I. J., Chen, C., Paw, B. H., Hamza, I. Iron and porphyrin trafficking in heme biogenesis. The Journal of biological chemistry 285, 26753-26759 (2010); Yuan, X., Fleming, M. D., Hamza, I. Heme transport and erythropoiesis. Current opinion in chemical biology 17, 204-211 (2013); Yi, L., Morgan, J. T., Ragsdale, S. W. Identification of a thiol/disulfide redox switch in the human BK channel that controls its affinity for heme and CO. The Journal of biological chemistry 285, 20117-20127 (2010)).

The NO-ferrohaem-dependent sGC activation mechanism takes place in vivo in whole eosinophils treated with azide or hydroxylamine. In this case, azide or hydroxylamine is metabolized by catalase to form NO-ferrohaem-catalase complex, which then transfer NO-ferrohaem group to sGC, without the release of NO. In this regard, it is likely that the blood vessel relaxant activities of azide and hydroxylamine are mediated by the NO-ferrohaem entity, but not by free NO. The involvement of the NO-ferrohaem transfer mechanism in erythrocyte- and myocardiocyte -mediated bioactivation of nitrite or nitroglycerin are further examples of the physiological mechanism underlying the present invention.

The invention, which targets the NO-haem signalling pathway(s), should have broad biomedical applications. Multiple and long-lasting attempts to use different NO-donor agents (as potential drugs) in the prior art were rather disappointed. For example, largest pharmaceutical companies and scientific agencies donated hundreds of millions of dollars for the development of new NO-based cardiovascular drugs, but none appeared on the market.

Another aspect of the present invention identifies the mobile sGC-activating NO-ferrohaem as an NOS-derived signalling species, which is supposedly present in biological fluids in pM to nM concentrations and may account for the paracrine and endocrine function of the constitutive NOS-1 and NOS-3. The recognition of NO-ferrohaem as a principal NOS-derived species opens new perspectives for personalized medicine, for early diagnostics and correction of health disorders. The invention is substantiated by the fact that a malfunctional NOS-sCG signalling is one of the earliest cause and sign of numerous diseases in humans, animals and plants. The present invention therefore provides a method(s) for the evaluation of the NOS/NO-haem/sGC pathway in biological samples, comprising the provision of a biological sample isolated from a human or animal body and determination of the presence and/or quantification of mobile NO-ferrohaem.

The mobile sGC-activating-NO-ferrohaem is ubiquitously generated in live cells by the NOS-containing multi-protein complex, as well as by some other enzymatic systems. As such, the mobile NO-ferrohaem may perform autocrine, paracrine and endocrine function, very much alike a hormone, and it can be detected in the extracellular medium (e.g. blood plasma). The evaluation of the NOS/NO-haem/sGC pathway in a biological sample therefore might be of diagnostic importance. The generation/supply of mobile NO-haem species might critically affect the sCG-dependent physiological functions, including regulation of systemic and regional blood flow, cardiac contraction, platelet adhesion/aggregation, gastro-intestinal motility, neural communications, neuroendocrine secretion, vision, penile erection, cell proliferation/differentiation and immunity. The determination of the mobile NO-ferrohaem levels in biological fluids and pharmaceutical correction of these levels comprise a novel approach for the diagnostics and treatment of many health disorders, caused by a compromised NOS/sGC pathway. In the prior art, numerous attempts to use different NO-gas detection methods for diagnostic purposes did not result in the development of valuable diagnostic tests.

A preferred method for the detection of NOS/NO-haem/sCG activity in a biological sample is carried out by the determination of NO-ferrohaem (a) blood sample under basal conditions; (b) blood sample pretreated with nitrite under deoxygenated conditions; (c) blood sample pretreated with an activator of heme synthesis, preferably with 5-aminolevulinic acid (alternatively, with glycine or protoporphyrin IX); (d) blood sample pretreated with an inhibitor of heme synthesis, preferably with the physiologically high concentration of glucose.

The activation of apo-sGC by endogenous NO-ferrohaem in a biological sample (preferably, blood) is determined by the following steps:
a) providing a sample containing nitrosylated ferrous heme (NO-ferrohaem),
b) adding an apo-(heme-free)-sGC probe, under conditions allowing the interaction of NO-ferrohaem with sGC,
c) measuring the sGC activity (cyclic GMP levels) cyclase (sGC).

The direct determination of mobile NO-ferohaem in a biological sample in preferably involves one or more of the following assays to be carried out:
(a) electron paramagnetic resonance (EPR)-based assay,
(b) fluorescence-based assay,
(c) luminescence-based assay,
(d) microelectrode (biochip)-based assay.

The present invention is not restricted to any of the above-mentioned methods, but can also involve other approaches that allow the direct determination of NO-ferrohaem, in particular in a biological fluid derived from the humans, animals or plants. In a preferred embodiment, mobile NO-ferrohaem is determined under basal conditions, wherein the blood is treated with nitrite in deoxygenated conditions followed by the separation of the blood plasma. The blood is then treated in vitro with an activator of haem synthesis, such as 5-aminolevinilic acid, followed by separation of plasma. Further examples of biological fluids are samples taken from the human or animal body such as blood, lymph, cerebral fluid, lacrimal fluid, saliva, urine, gut content, sweat, nasal secret, bronchial secret, sperm.

A preferred method for the determination of NO-ferrohaem in a biological sample comprises the visualization of NO-ferrohaem-responsive sGC/apo-sGC in cells and tissues using
a. fixation of cyclic GMP with formaldehyde,
b. labelled antibodies against the formaldehyde-fixed cyclic GMP.

A preferred method for the determination of NO-ferrohaem in the biological sample comprises the use of the apo-sGC probe and the determination of cyclic GMP by one of the following assays:
(a) Enzyme Linked Immunosorbent Assay (ELISA),
(b) Spin-immunoassay,
(c) Radio-immunoassay.

In a preferred embodiment, the direct measurement of the mobile (loosely-bound) NO-ferrohaem in the biological fluid is carried out by EPR-based assay which comprises, in a first step, the extraction of the loosely-bound NO-ferrohaem (e.g from the biological sample, such as blood plasma) using neutral acetone, or acidified acetone, or 2-butanone. In a second step, the NO-haem is concentrated in deoxygenated conditions, transferred to an appropriate solvent/solution, and the characteristic icon is registered.

A preferred method is EPR-based assay, which allows the specific NO-haem detection due to characteristic EPR signal.

A further preferred method comprises the application of a fluorescence-based assay, which comprises the elaboration of a specific fluorescent probe for NO-ferrohaem.

A further preferred method comprises the application of a microelectrode (biochip)-based assay, which comprises the elaboration of the biochip, in which NO-ferrohaem binding is transformed to the electric current.

Preferably, visualization of the distribution of NO-ferrohaem-responsive sGC in cells and tissues (e.g. brain or blood vessels) comprises the elaboration of immunohistochemistry-based methods and technologies using an antibody that is directed against formaldehyde-fixed cyclic GMP.

### Example:

In a preferred embodiment, the biological sample (venous blood) is obtained from a patient suffering from arterial hypertension, wherein the twice-lower than control the NO-ferrohaem level in blood imply the insufficiency of the NO-haem/sGC pathway in the circulation. The determination of NO-ferrohaem level in blood was performed by the direct EPR method.

Fresh venous blood (5 ml) was mixed with neutral acetone (10 ml), then the acetone-phase was removed and evaporated; the obtained sediment was dissolved in heptane and the EPR spectra was recorded at room temperature.

The present invention also describes diagnostic kits for the determination of mobile NO-ferrohaem in a biological sample, comprising an apo-sGC probe plus additives and means for the determination of cyclic GMP by ELISA, spin-immunoassay, or radio-immunoassay.

The invention further describes the use of nitrosylated haem (NO-ferrohaem) for the preparation of a biomarker used in the treatment or diagnosis of nitro oxidative stress or sGC-dependent physiological disorders.

Finally, the present invention describes a pharmaceutical composition, comprising a nitrosylated ferrous haem (NO-ferrohaem) and a pharmaceutically acceptable ligand bound to haem-iron at the 6^{th} position. Preferably, the NO-ferrohaem in a pharmaceutical composition is a ligand-bound NO-ferrohaem, such as: (NO)(OH-)haem, (NO)(HS-)haem, (NO)(CO)haem, (NO)(CN-)haem, (NO)(cysteine)haem, (NO)(L-arginine)haem, (NO)(histidine)haem.

The inventor synthesized the sGC-activating NO-ferrohaem preparations by the treatment of ferrohaem solution by NO-gas in deoxygenated conditions in the absence or in the presence of a corresponding ligand (such as albumin, heparin, H2S, cysteine, L-arginine etc.) followed by a removal of the unbound NO by vacuum in a Thumberg-type vessel.

Preferably, the NO-ferrohaem in the pharmaceutical composition is nitrosylated ferrous protoporphyrin IX. The sCG-activating mobile NO-ferrohaem can also bind a variety of commonly used drugs that are related to a malfunction of the NOS/sCG-signalling pathway. As such, the pharmaceutical composition comprises NO-ferrohaem and a pharmaceutically acceptable carrier for use in the treatment of cardiovascular disease, neurological disease, respiratory disease, immune disorder, eye disease, severe stress and/or premature aging.

Preferably, the sCG-activating NO-ferrohaem of the invention allow the prevention and/or treatment of a disease, wherein the cardiovascular disease is selected from the group consisting of endothelial dysfunction, atherosclerosis, arterial hypertension, pulmonary artery hypertension, heart failure, stroke, microcirculation disorders, ischemic heart disease, angina, arterial thrombosis, deep venous thrombosis, superficial venous thrombosis.

Preferably, the neurological disease is selected from the group consisting of Parkinson's, Alzheimer's, Huntington's diseases, multiple sclerosis, stroke, memory disorders, pain, erectile dysfunction, sleeping disorders, anxiety. The respiratory disease is preferably selected from the group consisting of asthma, bronchitis, chronic obstructive pulmonary disease (CPOD), and/or the immune disorder is preferably selected from the group consisting of sepsis, allergy, AIDS, or eye disease is selected from the group consisting of glaucoma, retinal degeneration and uveitis.

The detection of malfunctional NO-haem/sGC pathway in humans and animals should be followed by the monitoring and correction of the NO-haem levels through the direct NO-haem supply or through the stimulation of endogenous NO-haem production using behaviour, diet, pharmacological or genomic means.

The inventor has shown that intravenous injection of NO-ferrohaem solution to anesthetized rats (10 nmol/kg; bolus dose) results in the development of sGC-inhibitor-sensitive hypotension. These data suggest that synthetic NO-ferrohaem can reach sGC in the vasculature and provide the vasodilation. In the prior art, free NO induces transient vasodilation and hypotension.

It was also shown in the present invention that NO-ferrohaem also elicits the direct long-lasting vasodilating activity in isolated rat mesenteric arteries.

The mobile NO-ferrohaem species as described in the present invention also relates to its application in the development of active agents and/or methods for the prevention or treatment of platelet activation and thrombosis. The inventor has shown that NO-ferrohaem, at low nM concentrations efficiently inhibits the thromboxane-induced platelet aggregation in human platelet-rich plasma. In the prior art, free NO (or NO-donors) inhibits platelet aggregation, but only at µM concentrations. Note: In the prior art, NO-ferrohaem is considered as a sink for NO and thus should not activate sGG and elicit sGC-dependent bioactivity.

In summary, the labile (mobile) NO-ferrohaem has been found to be a novel endogenous regulator of the sGC-dependent physiological functions. The contribution of the S-nitrosative mechanism to the NO-haem bioactivity is also likely and should be specifically studied. The determination and pharmacological correction of the mobile NO-ferrohaem levels in biological fluids can help to treat many health disorders in humans and animals.

The mobile NO-ferrohaem as described in the present invention appears to be the optimal form of NO-related bioactivity which is generated enzymatically without participation of free NO. The NO-ferrohaem according to the invention has potential benefits over free NO, as it can activate both Fe2⁺-haem-sGC and apo-sGC. The NO-haem can be efficiently transported to a target and allow the compartmental and inter-compartmental signalling. In the prior art, the non-directional non-controlled diffusion, non-specific deleterious reactions and partitions into lipids, elicit NO as an unfortunate biological and pharmacological agent.

Furthermore, NO-ferrohaem according to the invention specifically interacts with sGC (H-NOX) whereas free NO comprises multiple non-specific interactions. NO-ferrohaem according to the invention is both highly efficient and suitable for a storage, whereas free NO is difficult to store. Free NO also inhibits Cyt C oxidase and results in non-specific nitrosation/nitration of protein residues, whereas the NO⁺Fe²⁺haem-carrier of the invention allows for targeted S-N-nitrosation of specific protein residues.

The invention is further illustrated in more detail in the following examples and figures. The NO-haem as used in the following examples and figures refers to nitric oxide (NO) ferro (Fe2⁺) - haem, i.e. mobile (labile) NO-ferrohaem.

### Material and Methods:

### Preparation of NO-ferrohaem complex

NO-haem complex was prepared as 10mM stock solution in DMSO by (1) reaction of haemin (ferrihaem) with the nitroxyl-donor, Angeli's salt; or (2) reduction of ferrihaem by sodium dithionite and the following treatment of obtained ferrohaem by NO-gas in the Thumberg-type vessel in deoxygenated conditions. EPR spectra confirmed the quantitative conversion of ferrihaem to NO-ferrohaem. The 100 µL aliquots of the 10 mM NO-ferrohaem solution were frozen in liquid nitrogen. Before the experiment the 10mM NO-ferrohaem solution was dissolved in 0.05N NaOH to obtain 1mM NO-ferrohaem solution. Further sequential dilutions were done with the physiological phosphate buffer.

### Platelet studies

Human venous blood was collected into a plastic polypropylene syringe containing sodium citrate. The donor of blood did not receive any medication for at least 10 days. Platelet-rich-plasma (PRP) was prepared by centrifugation of the blood at 180g for 10 minutes at room temperature. The cloudy supernatant containing platelets was carefully removed with a pipette and transferred into a clean polypropylene tube and capped. Platelets were used for the experiments within 3 hr after blood collection. Platelet function studies were performed using a Dual Channel Aggregometer (Chronolog, Havertown). The PRP was adjusted to a platelet count of 200-300x 106/ml. Platelet aggregation was induced by the addition of a synthetic thromboxane A₂ receptor agonist, U46619 (Cayman Chemical, Ann Arbor). PRP was placed in the aggregometer cuvette and continuously stirred at the rate of 1000 rpm. Platelet aggregation was determined by measuring the change in the optical density after addition of 1µM U46619. Different concentrations of NO-ferrohaem were added to the PRP suspensions 1 min before addition of U46619.

### Isometric tension studies

For the pilot experiment a male Swiss mouse (35 g) was sacrificed under ether anaesthesia. Femoral artery was isolated and cleaned from the adhering fat and connective tissue. Artery rings (2 mm) with endothelium were mounted in the organ chambers filled with Krebs solution continuously bubbled with 95% 02/ 5% C02 at 37°C. The composition of the solution was as following: 119 mM NaCl, 4.7 mM KCI, 14.9 mM NaHC03, 1.2 mM MgSO4.7H2O, 2.5 mM CaCI2, 1.18 mM KH2PO4, and 5.5 mM glucose; pH 7.4. Vascular reactivity was measured by the monitoring the changes in isometric tension using a wire myograph (Danish Myo Technology, Arhus, Denmark). After pre-contraction of arterial rings with 1µM 5-HT (serotonin; Sigma-Aldrich), the cumulative concentrations of NO-ferrohaem were added. In the end of experiment, an inhibitor of the soluble guanylate cyclase (ODQ; 1µM) was added.

### Blood pressure studies

For the pilot experiment, a male Wistar rat weighing 350g from Janvier Labs (Le Genest-Saint-Isle, France) was used. Rat was anaesthetised with isoflurane 1-2% (Baxter S.A.S, Maurepas, France) and analgesia was ensured by sufentanil (Mylan, Pittsburgh PA, USA) (0.1 µg/kg of body weight) administered subcutaneously. Animal was tracheotomised and lungs ventilated throughout the experiment. The ventilator (Harvard Rodent Ventilator 683, Harvard Instruments, South Natick, MA, USA) was set to maintain a PaCO2 of 40 mmHg and oxygen was added to maintain a PaO2 of 100 mmHg. The left femoral artery was used to measure mean arterial pressure (MAP) and heart rate (HR). The rectal temperature was continuously monitored. The NO-ferrohaem was injected at dose 2µmol/kg (s.c.) before and after the injection of methylene blue (1 µmol/kg; s.c.). In the end of experiment rat was euthanized using the lethal dose of Pentothal (100 mg/kg; i.v.).

### Figure Legends:

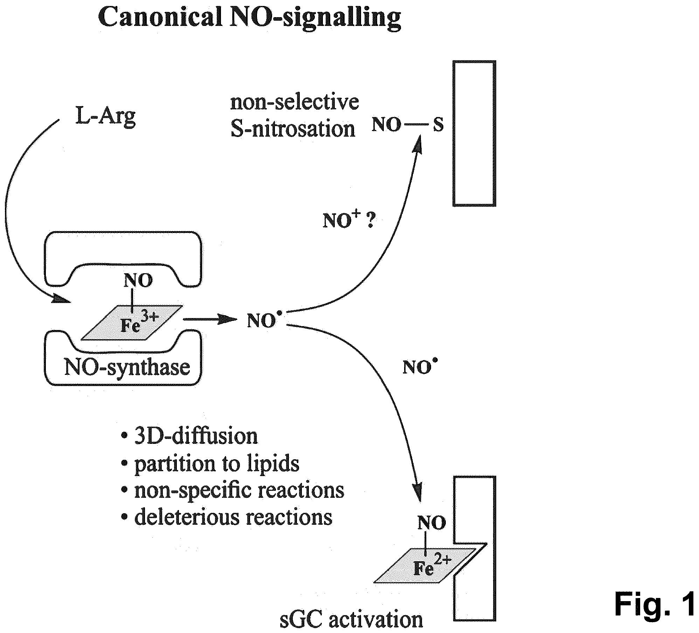

Fig. 1: The NO signalling paradigm as known from the prior art. The NO gas produced from the amino acid, L-arginine (L-Arg) by the haem-containing enzyme(s) called NO-synthase, freely diffuses through the (inter)cellular space to bind the ferro(Fe²⁺)haem prosthetic group of the key intracellular regulatory enzyme, soluble guanylyl cyclase (sGC); the active ternary NO-ferrohaem-sGC complex produces the second messenger, cyclic GMP, which initiates the cascade of critical biochemical events. The NO is also involved in another important (sGC-independent) signalling mechanism, S-nitrosative modification of proteins; however, the mechanism of selective protein S-nitrosation is unknown. The non-directional diffusion of NO in cell, multiple non-specific and often deleterious NO reactions are not compatible with the nature's expediency principle.

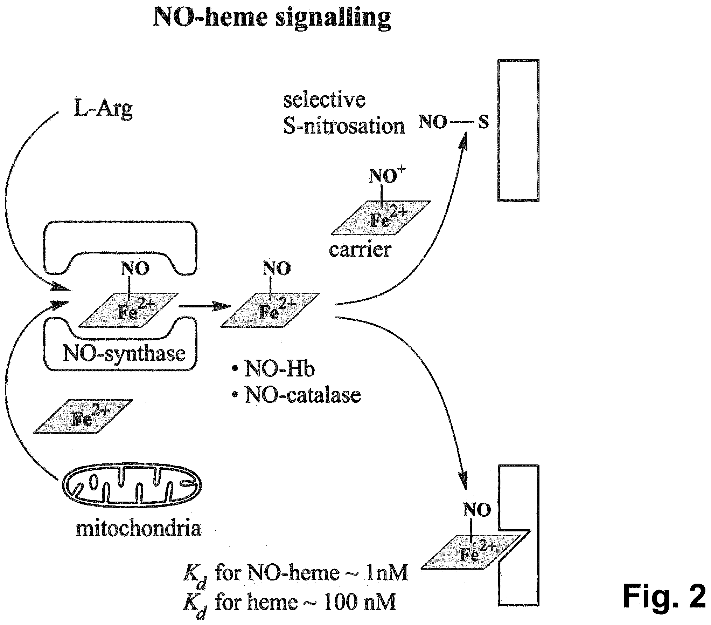

Fig. 2: The NO-ferrohaem signalling pathway according to the present invention. The NO-ferroheme, formed within NO-synthase, is replaced by fresh haem from the regulatory pool. In whole cell, a major product of NOS activity is NO-ferro(Fe²⁺)heme. Other potential sources of NO-ferroheme are NO-haemoglobin (NO-Hb), NO-catalase etc. The mobility, safety and high affinity for the soluble guanylyl cyclase (sGC) are prerequisites for the NO-ferrohaem being a major sGC-activating signalling species. Additionally, the NO-ferrohaem one-electron oxidation product, NO-ferrihaem, has a potential to mediate the selective S-nitrosative modification of proteins.

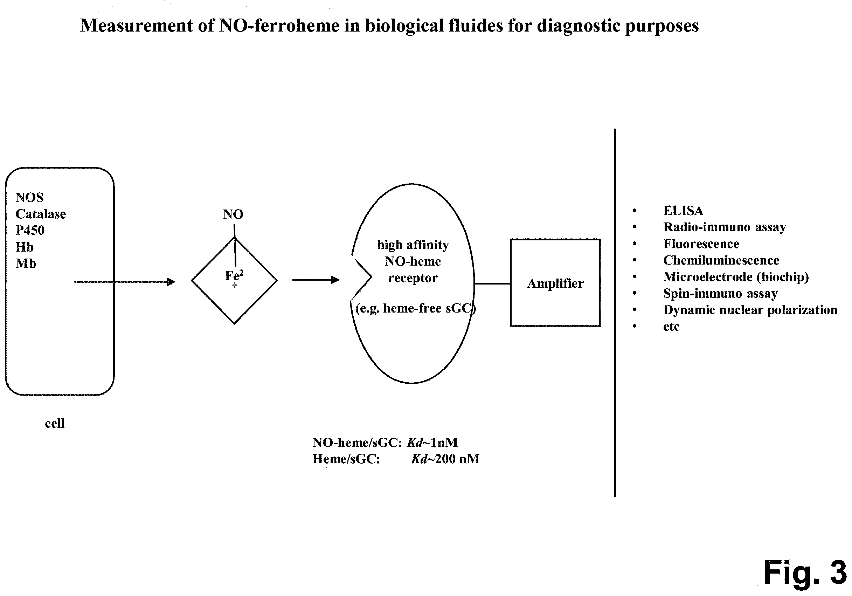

Fig. 3: Measurement of NO-ferrohaem in biological fluids for diagnostic purposes

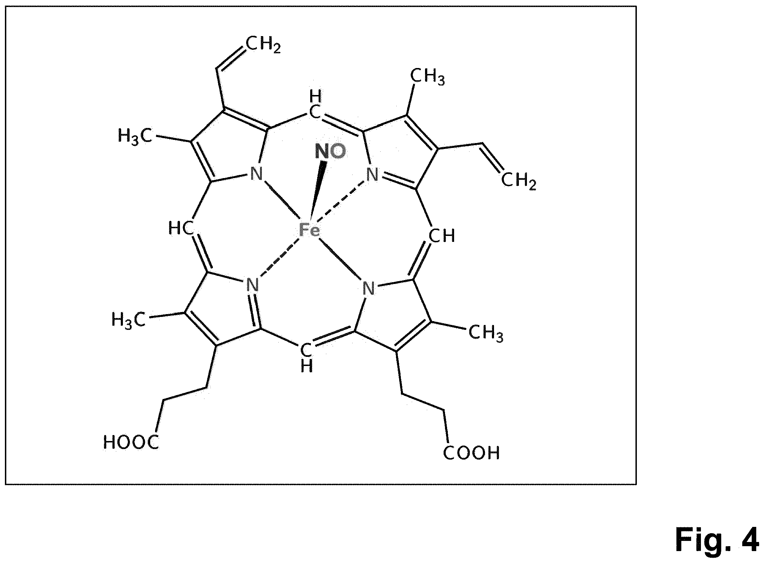

Fig. 4: Chemical structure of NO-ferrohaem complex

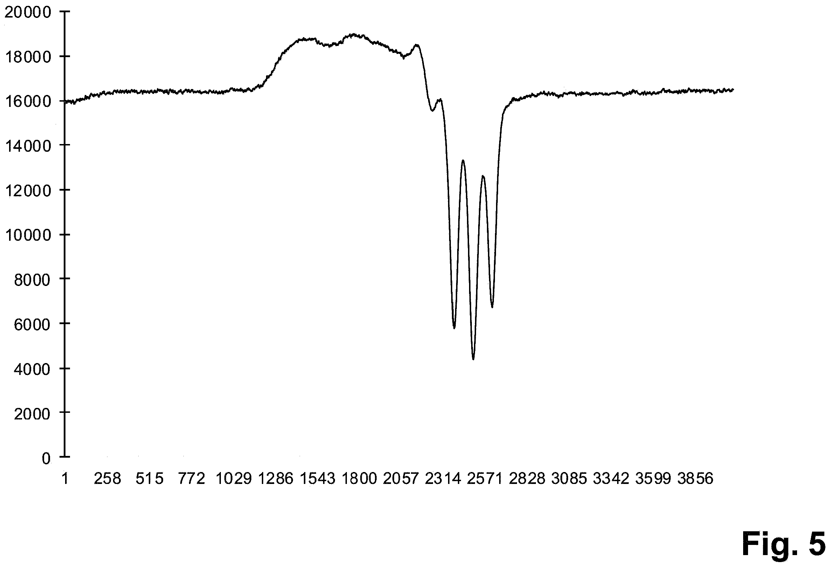

Fig. 5: EPR spectrum on NO-ferrohaem complex (1mM solution). EPR spectrum was recorded using an X-band radiospectrometer MS200 (Magnettech, GmbH). Instrument parameters: microwave power 10mW, modulation amplitude 1gauss, modulation frequency 100 kGz, center fiels 3655 gauss, sweep field 400 gauss, T=77K.

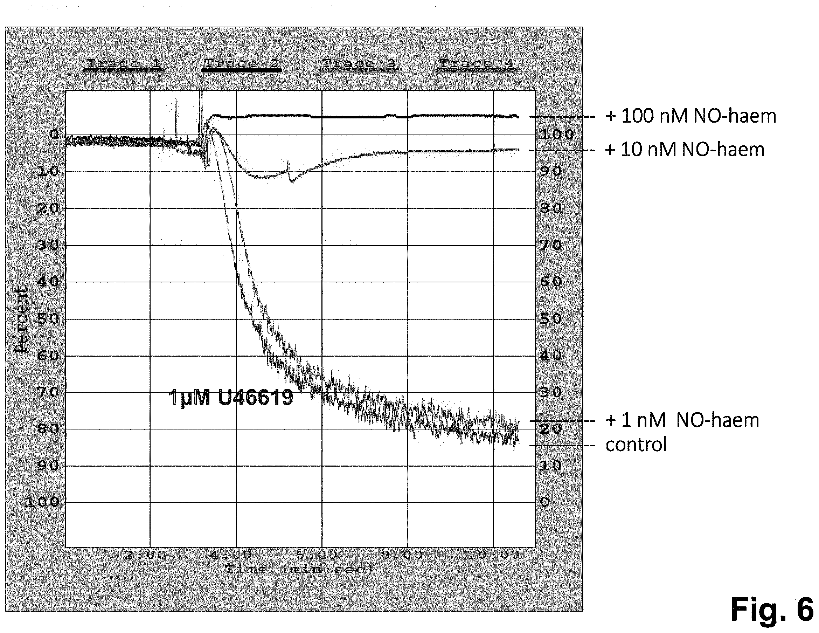

Fig. 6: NO-ferrohaem inhibits of the U46619-induced platelet aggregation in human platelet-rich plasma (PRP) preparation. Addition of 1µM U46619 to PRP triggered the platelet aggregation (control, blue trace). NO-haem added to PRP (at 1nM, 10nM or 100nM; 30 seconds before U46619) concentration-dependently prevented the aggregation (green, red and black traces). Measurements performed together with Malak Abbas and Valerie Schini-Kerth, University of Strasbourg, France.

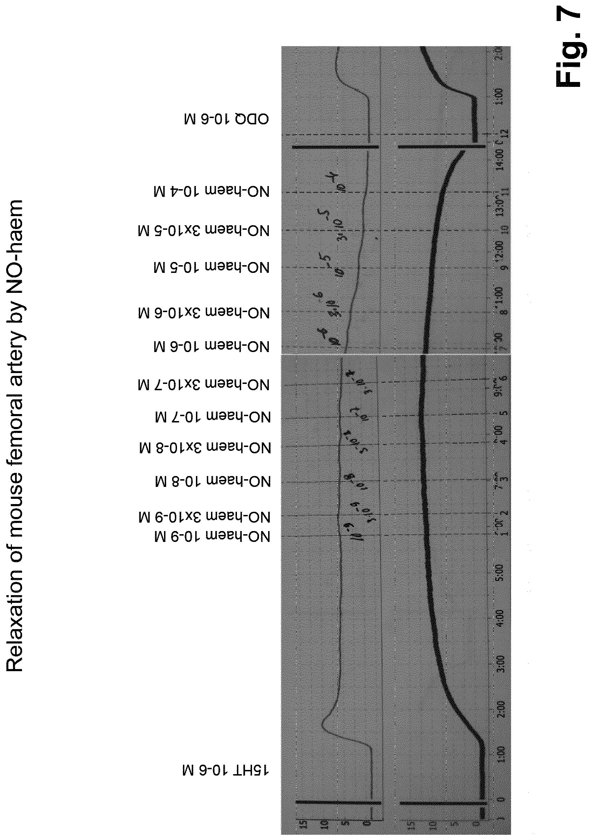

Fig. 7: Vasodilating activity of NO-ferrohaem in the isolated 15HT-precontracted mouse femoral artery. Mouse femoral artery rings (2 mm long) were isolated and mounted in the organ chambers filled with Krebs solution (37°C; 95% O2/5% CO2). Isometric tension studies performed by standard method. Addition of NO-ferrohaem to the 15HT (1µM)-pre-contracted arteries, produced a concentration-dependent relaxation (in 100nM-100µM concentration range). The inhibitor of the soluble guanylate cyclase, ODQ (1µM) completely reversed the NO-ferrohaem-induced relaxation. Measurements performed together with Brigitte Pollet and Valerie Schini-Kerth, University of Strasbourg, France.

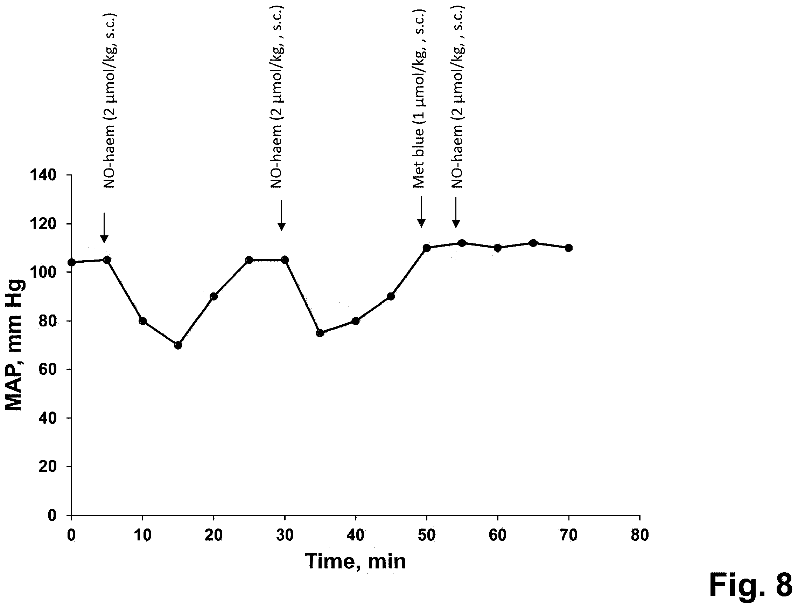

Fig. 8: Hypotensive activity of NO-ferrohaem in the anesthetized Wistar rat. Bolus subcutaneous injection of NO-ferrohaem (2µmol/kg; 0.4ml) resulted in the development of transient (about 15 min) arterial hypotension. The injection of the inhibitor of the soluble guanylate cyclase, methylene blue prevented this NO-ferrohaem activity. Measurements performed together with Melanie Burban, University of Strasbourg, France.

## Claims

1. An in-vitro method for the detection of a malfunction of the nitric oxide synthase (NOS)/soluble guanylyl cyclase (sGC) pathway in a biological sample, comprising the provision of a biological sample that has been isolated from a human or animal body and determination of the presence and/or quantity of mobile (loosely-bound) nitrosylated ferrous haem (NO-ferrohaem) in the isolated biological sample by determining the activation of haem-free sGC (apo-sGC) by endogenous NO-ferrohaem in the biological sample, wherein the biological activity of apo-sGC in the biological sample is determined by the steps:
a. providing the sample containing nitrosylated ferrous haem (NO-ferrohaem),
b. adding an apo-(haem-free)-sGC probe to the sample, under conditions allowing the interaction of NO-ferrohaem with sGC,
c. measuring the levels of cyclic GMP(cGMP) levels to determine the activity of soluble guanylyl cyclase (sGC) in the presence of NO-ferrohaem.

2. The method according to claim 1, wherein the determination of nitrosylated ferrous haem (NO-ferrohaem) is carried out (a) blood sample under basal conditions; (b) with the blood sample pretreated with nitrite under deoxygenated conditions; (c) with the blood sample pretreated with an activator of haem synthesis, preferably 5-amino levulinic acid; (d) blood sample pretreated with an inhibitor of haem synthesis, preferably with physiologically high concentration of glucose.

3. The method according to claim 1, wherein the determination of nitrosylated ferrous haem (NO-ferrohaem) in the biological sample involves one or more of the following assays to be carried out:
a. electron paramagnetic resonance (EPR)-based assay,
b. luminescence- or fluorescence based assay,
c. microelectrode (biochip)-based assay.

4. The method according to claim 1, wherein the method further comprises the visualization of NO-ferrohaem-responsive soluble guanylyl cyclase (sGC) in fluid, cell(s), tissue or vessel(s) of the biological sample by
a. fixing cyclic GMP (cGMP) with formaldehyde,
b. directing labelled antibodies against the formaldehyde-fixed cGMP.

5. The method according to claim 1, wherein the determination of nitrosylated ferrous haem (NO-ferrohaem) in the biological sample comprises the use of the apo-sGC-probe and the determination of cyclic GMP (cGMP) by one or more of the following assays: (a) Enzyme Linked Immunosorbent Assay (ELISA), (b) Spin-immunoassay, (c) Radio-immunoassay.

6. The method according to anyone of claims 1 to 5, wherein the biological sample is taken from blood, lymph, cerebral fluid, lacrimal fluid, saliva, urine, gut content, sweat, nasal secret, bronchial secret, sperm.

## Patentansprüche

1. In-vitro-Verfahren zum Nachweis einer Fehlfunktion des Stickstoffmonoxid-Synthase (NOS)/lösliche Guanylylcyclase (sGC)-Wegs in einer biologischen Probe, umfassend
die Bereitstellung einer aus einem menschlichen oder tierischen Körper isolierten biologischen Probe und die Bestimmung des Vorhandenseins und/oder der Menge von mobilem (lose gebundenem) nitrosyliertem eisenhaltigem Häm (NO-Ferrohaem) in der isolierten biologischen Probe durch Bestimmung der Aktivierung von Hämfreiem sGC (apo-sGC) durch endogenes NO-Ferrohaem in der biologischen Probe, wobei die biologische Aktivität von apo-sGC in der biologischen Probe durch die folgenden Schritte bestimmt wird:
a. Bereitstellung der Probe, die nitrosyliertes eisenhaltiges Häm (NO-Ferrohaem) enthält,
b. Zugabe einer apo-(Häm-freien)-sGC-Sonde zu der Probe, unter Bedingungen, welche die Wechselwirkung von NO-Ferrohaem mit sGC erlauben,
c. Messung der Spiegel von zyklischem GMP (cGMP) zur Bestimmung der Aktivität der löslichen Guanylylzyklase (sGC) in Gegenwart von NO-Ferrohaem.

2. Verfahren nach Anspruch 1, wobei die Bestimmung von nitrosyliertem eisenhaltigem Häm (NO-Ferrohaem) durchgeführt wird
(a) mit einer Blutprobe unter basalen Bedingungen;
(b) mit der mit Nitrit vorbehandelten Blutprobe unter desoxygenierten Bedingungen;
(c) mit der mit einem Aktivator der Häm-Synthese, vorzugsweise 5-Aminolävulinsäure, vorbehandelten Blutprobe;
(d) mit der mit einem Inhibitor der Häm-Synthese, vorzugsweise mit einer physiologisch hohen Konzentration von Glucose, vorbehandelten Blutprobe.

3. Verfahren nach Anspruch 1, wobei die Bestimmung von nitrosyliertem eisenhaltigem Häm (NO-Ferrohaem) in der biologischen Probe einen oder mehrere der folgenden durchzuführenden Assays umfasst:
a. Elektronenspinresonanz (ESR)-basierter Assay,
b. Lumineszenz- oder Fluoreszenz-basierter Assay,
c. Mikroelektroden (Biochip)-basierter Assay.

4. Verfahren nach Anspruch 1, wobei das Verfahren ferner die Visualisierung von NO-Ferrohaem-reaktiver löslicher Guanylylcyclase (sGC) in Fluid, Zelle(n), Gewebe oder Gefäß(en) der biologischen Probe umfasst, indem
a. zyklisches GMP (cGMP) mit Formaldehyd fixiert wird,
b. markierte Antikörper gegen das Formaldehyd-fixierte cGMP gerichtet werden.

5. Verfahren nach Anspruch 1, wobei die Bestimmung von nitrosyliertem eisenhaltigem Häm (NO-Ferrohaem) in der biologischen Probe die Verwendung der apo-sGC-Sonde und die Bestimmung von zyklischem GMP (cGMP) durch einen oder mehrere der folgenden Assays umfasst: (a) Enzyme-Iinked Immunosorbent Assay (ELISA), (b) Spin-Immunoassay, (c) Radio-Immunoassay.

6. Verfahren nach einem der Ansprüche 1 bis 5, wobei die biologische Probe aus Blut, Lymphe, Hirnflüssigkeit, Tränenflüssigkeit, Speichel, Urin, Darminhalt, Schweiß, Nasensekret, Bronchialsekret, Sperma gewonnen wird.

## Revendications

1. Procédé in vitro pour la détection d'un dysfonctionnement de la voie oxyde nitrique synthase (NOS)/guanylyl cyclase soluble (sGC) dans un échantillon biologique, comprenant la fourniture d'un échantillon biologique qui a été isolé à partir du corps d'un être humain ou d'un animal et la détermination de la présence et/ou de la quantité d'hème ferreux nitrosylé (NO-ferrohème) (faiblement lié) mobile dans l'échantillon biologique isolé en déterminant l'activation de la sGC exempte d'hème (apo-sGC) par le NO-ferrohème endogène dans l'échantillon biologique, dans lequel l'activité biologique de l'apo-sGC dans l'échantillon biologique est déterminée au moyen des étapes qui suivent :
a. la fourniture de l'échantillon qui contient l'hème ferreux nitrosylé (NO-ferrohème) ;
b. l'ajout d'une sonde apo-sGC-(exempte d'hème) à l'échantillon, sous des conditions qui permettent l'interaction du NO-ferrohème avec la sGC ;
c. la mesure des niveaux de la GMP cyclique (cGMP) afin de déterminer l'activité de la guanylyl cyclase soluble (sGC) en présence de NO-ferrohème.

2. Procédé selon la revendication 1, dans lequel la détermination d'hème ferreux nitrosylé (NO-ferrohème) est mise en œuvre (a) à partir d'un échantillon de sang sous des conditions basales ; (b) alors que l'échantillon de sang est prétraité à l'aide de nitrite sous des conditions désoxygénées ; (c) alors que l'échantillon de sang est prétraité à l'aide d'un activateur de synthèse d'hème, de préférence l'acide 5-amino lévulinique ; (d) alors que l'échantillon de sang est prétraité à l'aide d'un inhibiteur de la synthèse d'hème, de préférence selon une concentration de glucose physiologiquement élevée.

3. Procédé selon la revendication 1, dans lequel la détermination d'hème ferreux nitrosylé (NO-ferrohème) dans l'échantillon biologique implique la mise en œuvre d'un ou de plusieurs des essais qui suivent :
a. un essai basé sur la résonance paramagnétique électronique (EPR) ;
b. un essai basé sur la luminescence ou la fluorescence ;
c. un essai basé sur une/des microélectrode(s) (biopuce).

4. Procédé selon la revendication 1, dans lequel le procédé comprend en outre la visualisation de la guanylyl cyclase soluble (sGC) sensible au NO-ferrohème dans un fluide, une/des cellule(s), un tissu ou un/des vaisseau(x) de l'échantillon biologique en :
a. fixant la GMP cyclique (cGMP) à l'aide de formaldéhyde ;
b. dirigeant des anticorps étiquetés à l'encontre de la cGMP fixée par formaldéhyde.

5. Procédé selon la revendication 1, dans lequel la détermination d'hème ferreux nitrosylé (NO-ferrohème) dans l'échantillon biologique comprend l'utilisation de la sonde apo-sGC et la détermination de la GMP cyclique (cGMP) au moyen d'un ou de plusieurs des essais qui suivent : (a) un essai d'immuno-absorption enzymatique (ELISA), (b) un essai spin-immunologique et (c) un essai radio-immunologique.

6. Procédé selon l'une quelconque des revendications 1 à 5, dans lequel l'échantillon biologique est prélevé à partir du sang, de la lymphe, du liquide cérébral, du liquide lacrymal, de la salive, de l'urine, du contenu stomacal, de la sueur, des sécrétions nasales, des sécrétions bronchiques ou du sperme.
